# EUROPEAN PATENT SPECIFICATION

(11) **EP 3 166 501 B1**
(45) Date of publication and mention of the grant of the patent: **16.08.2023**
(21) Application number: 15819387.0
(22) Date of filing: 24.06.2015
(51) Int. Cl.: A61B 8/12, A61M 25/01, A61M 25/09, A61B 8/06, A61B 8/08, A61B 8/00

(54) **SYSTEM FOR MEASURING FLUIDICS IN ARTERIES**
SYSTEM ZUR MESSUNG DER FLUIDIK IN ARTERIEN
SYSTÈME DE MESURE DE FLUIDIQUE DANS LES ARTÈRES

(30) Priority: 08.07.2014 US 201414326034; 13.08.2014 US 201462036841 P
(43) Date of publication of application: 17.05.2017
(73) Proprietor: Visveshwara, Nadarasa, Fresno, CA 93771 (US)
(72) Inventor: Visveshwara, Nadarasa, Fresno, CA 93771 (US)
(74) Representative: Marks & Clerk LLP
(86) International application number: PCT/US2015/037347
(87) International publication number: WO 2016/007288

(56) References cited:
- EP-A1- 0 611 310
- EP-A2- 0 253 687
- US-A- 4 665 925
- US-A- 5 174 295
- US-A- 5 246 007
- US-A- 5 405 318
- US-A- 5 433 205
- US-A1- 2014 005 706

## Description

### BACKGROUND OF THE INVENTION

This invention relates in general to systems and methods for measuring arteries and in particular to systems and methods for measuring parameters related to blood in arteries.

Conventional methods for measuring arterial blood mean velocity include Doppler echocardiography and Cardiac Catheterization. Non Invasive Doppler echocardiography used for measuring blood mean velocity in arteries employs a Doppler transducer outside the arteries to transmit ultrasound to the
arteries, and must be conducted so that the sound waves are directed to the arteries at angles not more than about 15 degrees from the direction of blood flow. Otherwise, the blood mean velocity measurement is not accurate. For certain locations of the human body, this may not be practical. Conventional cardiac catheterization cannot provide continuous measurement of cardiac output. It is therefore desirable to
provide improved systems and methods for measuring arterial blood mean velocity and cardiac output with related derived indices of cardiac function.

United States Patent Application US 5174295 A discloses a guide wire incorporating a system for measuring a characteristic flow of liquid in a vessel of a patient using a transducer positioned in the vessel.

### SUMMARY OF THE INVENTION

One embodiment of the invention is directed to an apparatus for performing measurements in an artery according to claim 1.

The application also describes a method for measuring blood mean velocity in an artery, which is not part of the present invention, comprising inserting into the artery an elongated catheter body having a proximal end and a distal end so that the distal end is located at a desired location in the artery for measuring blood mean velocity, the body defining therein a conduit therein. Thereafter inserted into the conduit at the proximal end of the body is a Doppler catheter that includes a Doppler transducer and a wire connected to the Doppler transducer so that the Doppler transducer passes through and extends outside the conduit to be located at said desired location. Ultrasound measurement of the blood mean velocity in the artery at said desired location using the Doppler transducer is performed.

The application also describes an apparatus for performing measurements in an artery, which is not part of the present invention, comprising a Doppler catheter which includes a Doppler transducer, a second transducer and wires connected to the Doppler transducer and the second transducer. The Doppler catheter is suitable for being inserted and withdrawn from a conduit in an elongated catheter body having the conduit therein for housing the wire of the Doppler catheter. The body has a proximal end and a distal end; wherein said wires are movable in the conduit relative to the catheter body so that the Doppler transducer, the second transducer and the wires are capable of being threaded into said conduit at the proximal end after the distal end of the catheter has been inserted into the artery, until the Doppler transducer and the second transducer emerge outside the conduit at the distal end of said body for performing ultrasound measurement in and measurement of a cross-sectional dimension of the artery.

### BRIEF DESCRIPTION OF THE DRAWINGS

Identical components are labeled by the same numerals in this document.
Fig 1A is a perspective view of a cardiac output Doppler catheter and an artery catheter to illustrate one embodiment of the invention.
Fig. 1B is an exploded view of portions of Fig. 1A within the circle 1B in Fig. 1A.
Fig. 2 is a perspective view of the cardiac output Doppler catheter of Fig. 1A where the Doppler transducer extends through a Y shaped connector and inserted into the artery catheter.
Fig. 3 is a cross-sectional view of one end of the Doppler catheter that includes the Doppler transducer and a cap for protecting the Doppler transducer.
Fig. 4 is a cross-sectional view of the Y shaped connector of Figs. 1B and of the proximal end of the artery catheter connected to the Y shaped connector.
Fig. 5 is a cross-sectional view of the Y shaped connector of Figs. 1B and one portion of the Doppler catheter that includes the Doppler transducer, a wire and a portion of a sleeve.
Fig. 6A is a partly perspective and partly cross-sectional view of the cardiac output Doppler catheter and artery catheter of Fig. 1A and a connector with a port connected to a syringe and another port for connection to a pressure transducer to illustrate applications of one embodiment of the invention.
Fig. 6B is an exploded view of a portion of the Doppler catheter and artery catheter of Fig. 6A within the circle 6B in Fig. 6A.
Fig. 7A is a perspective view of components of a portion of a cardiac output Doppler catheter to illustrate an alternative embodiment of the invention with two transducers.
Fig. 7B is a cross-sectional view of components of a portion of a cardiac output Doppler catheter of Fig. 7A after the components are assembled.
Fig. 7C is a cross-sectional view of a portion of the cardiac output Doppler catheter of Fig. 7A, 7B within the circle 7C in Fig. 7B to illustrate the connections of power and ground leads to the Doppler transducer.
Fig. 7D is a cross-sectional view of a portion of the cardiac output Doppler catheter of Figs. 7A, 7B within the circle 7D in Fig. 7B to illustrate the connections of power and ground leads to the transducer.
Fig. 7E is a perspective view of components of a portion of a cardiac output Doppler catheter of Fig. 7A after the components are assembled.
Fig. 8 is a schematic view of parts of the human body, including the heart and lung. This schematic also shows the positions of Doppler-tipped catheters and shows a computer, which can be used for determining the blood flow in a portion of the body.
Fig. 9 is a partly perspective and cut away view of the distal end of a Doppler catheter 300 to illustrate another alternative embodiment to the embodiments in the Parent Application.
Fig. 10 is a cross-sectional view of Doppler catheter 300 of Fig. 9.
Fig. 11 is a perspective view of one end of the Doppler catheter 300, showing the open lumen.
Fig. 12 is a perspective view of one end of the Doppler catheter 330 and artery catheter 350, showing the Doppler catheter at an off-center position relative to the artery catheter 350.
Fig. 13 is a cross-sectional view of the Doppler catheter 330 and artery catheter 350 of Fig. 12.

### DETAILED DESCRIPTION OF EXEMPLARY EMBODIMENTS

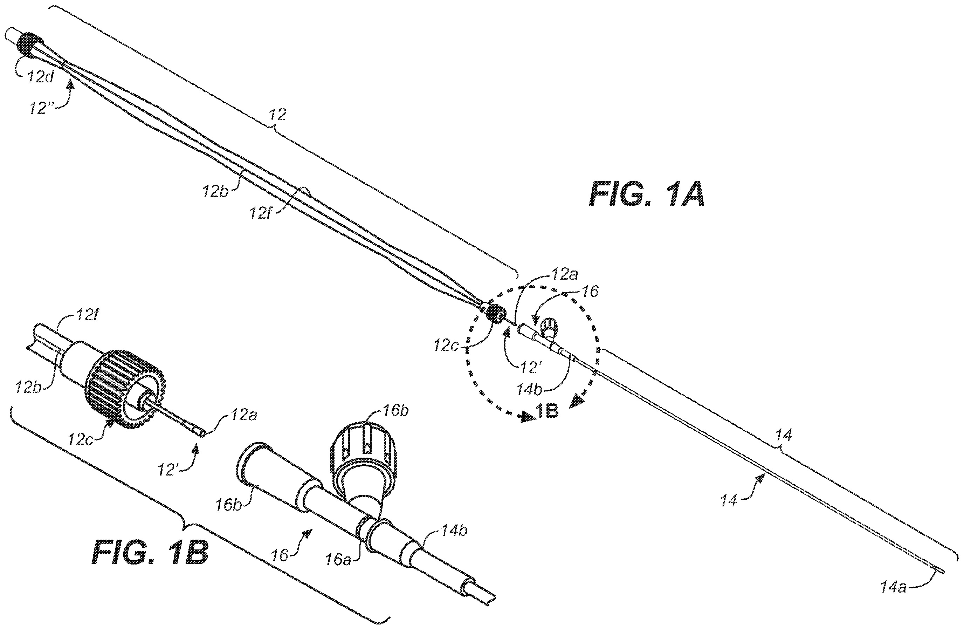

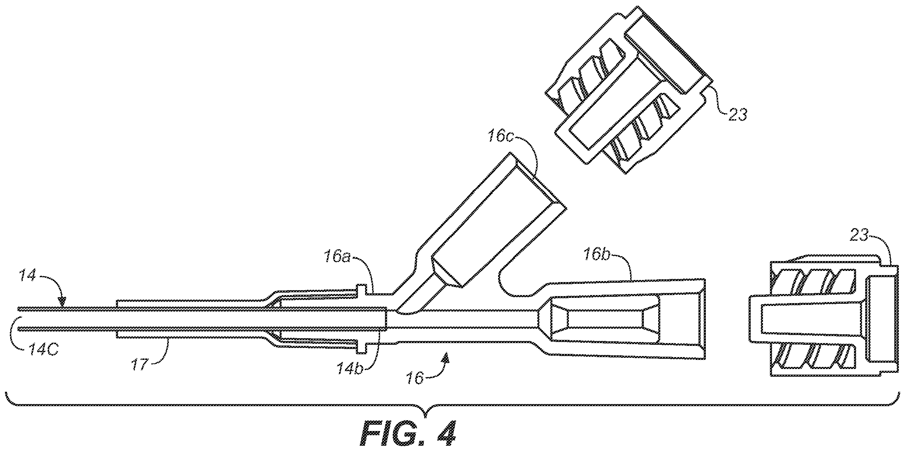

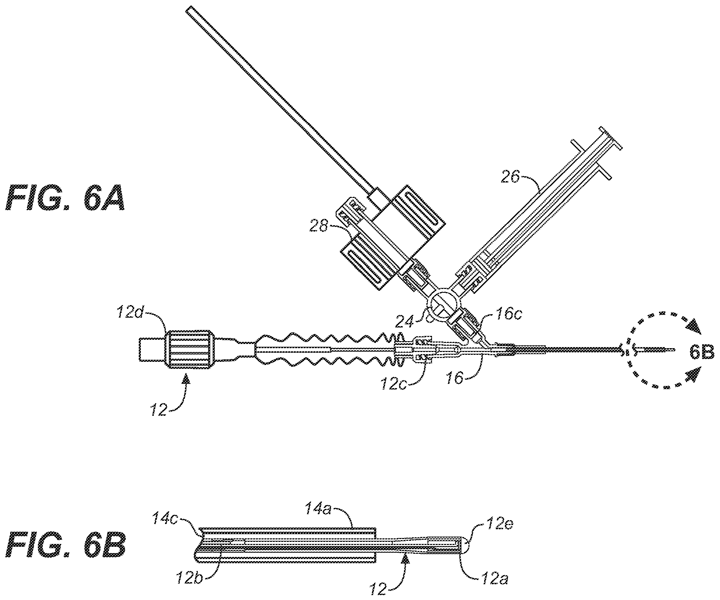

Fig 1A is a perspective view of a cardiac output Doppler catheter 12 and an artery catheter 14 to illustrate one embodiment of the invention. Fig. 1B is an exploded perspective view of portions of the apparatus in Fig. 1A within the circle 1B in Fig. 1A. Different from conventional Doppler echocardiography for measuring blood mean velocity which sends ultrasound waves from locations outside the human body, the cardiac output Doppler catheter of Fig. 1A is intended to be placed within an artery. In one application of the embodiment of Fig. 1A, the distal end 14a of an artery catheter 14 is first inserted into an artery (not shown) until its distal end 14a is located at or near a location in the artery desirable for performing ultrasound measurements. Then the distal end 12' of the cardiac output Doppler catheter 12 that contains a Doppler transducer 12a and an electrical wire 12b connected thereto are inserted at the proximal end 14b of the artery catheter into a conduit 14c (see Figs. 4, 6B) in the body of the artery catheter, where the insertion causes the Doppler transducer 12a and the electrical wire 12b to be moved relative to the conduit until the Doppler transducer 12a exits the distal end 14a of the artery catheter and is located at the desired location in the artery for performing ultrasound measurements.

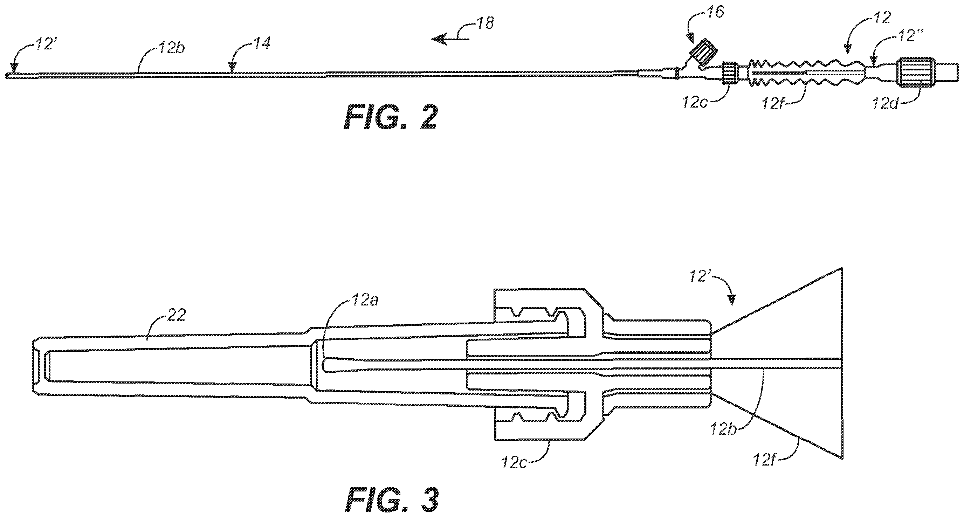

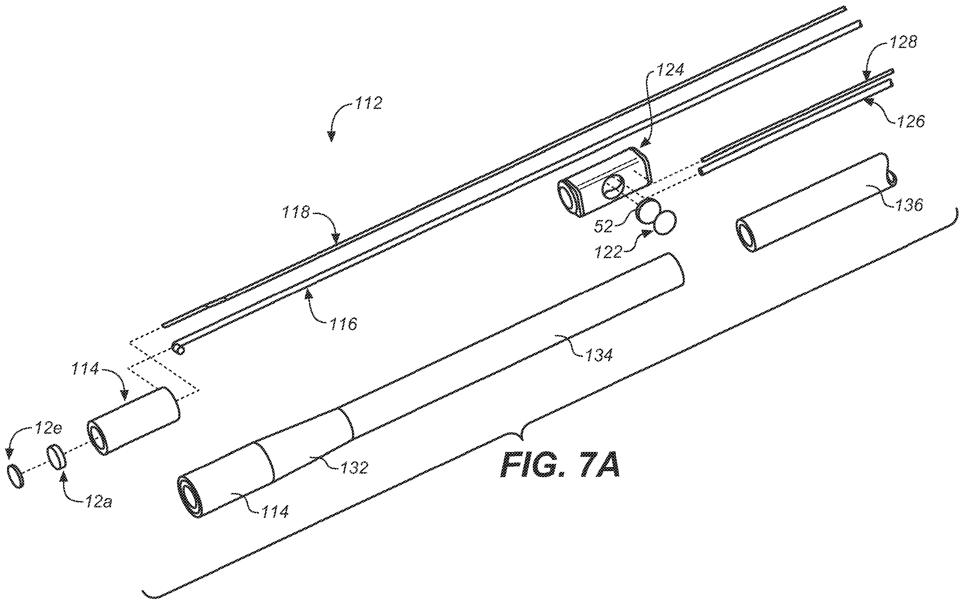

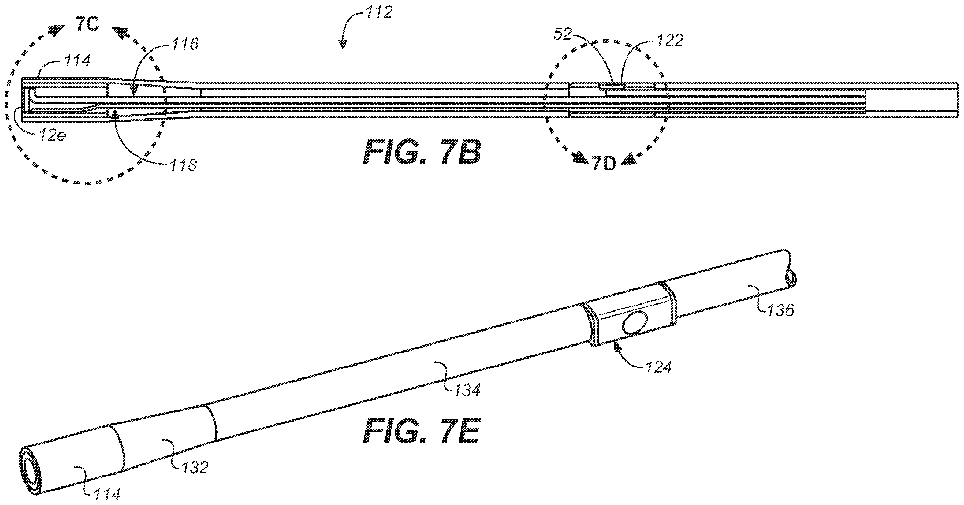

Ultrasound measurements are then performed in a conventional manner using the Doppler transducer 12a. Fig. 2 is a perspective view of the cardiac output Doppler catheter 12 of Fig. 1A where the distal end 12' of the Doppler catheter 12 containing Doppler transducer 12a is inserted through a preferably Y shaped connector 16 into the artery catheter. Fig. 6B shows the relative positions of the Doppler transducer 12a and the distal end 14a of the artery catheter when the Doppler transducer 12a is placed at a position suitable for performing ultrasound measurements in the artery. As shown in Fig. 6B, wire 12b of the cardiac output Doppler catheter 12 is inside the conduit 14c of the artery catheter 14, with a portion of the wire and the Doppler transducer 12a being outside of the conduit 14c to be placed at a location in the artery suitable for performing ultrasound measurement of blood mean velocity. The Doppler transducer 12a is protected by a Doppler tip layer 12e shown more clearly in Fig. 7A.

The advantage of the above arrangement is that since the Doppler transducer 12a is placed in the artery it is measuring, the Doppler transducer can be positioned to send ultrasound waves in a direction substantially parallel to the blood flow and this greatly improves the accuracy of the ultrasound measurement of blood mean velocity in the artery. Another advantage is that continuous measurement of cardiac output can be provided.

As shown in Figs. 1A and 1B, a first port 16a at the distal end of a Y shaped connector 16 is attached to the artery catheter 14 at the proximal end 14b of the artery catheter. As shown in Fig. 2, the distal end 12' of catheter 12 (containing Doppler transducer 12a) and the wire 12b attached to the transducer are threaded through the Y shaped connector and inserted into a conduit (shown in Figs. 4 and 6B) within the body of artery catheter 14. As shown in Figs. 1A and 1B, the cardiac output Doppler catheter 12 includes a connector 12c away from the proximal end 12" of the Doppler catheter 12, and another connector 12d at its proximal end 12". The cardiac output Doppler catheter 12 also includes a sleeve 12f enclosing and protecting the wire 12b from the environment. The wire 12b is movable relative to connector 12c, so that when the wire is moved from its position substantially all enclosed by sleeve 12f as shown in Fig. 1A to a position as shown in Fig. 2 where most of the wire 12b extends outside the sleeve and is inserted into the conduit of the artery catheter 14, the distal end of the wire 12b (and transducer 12a) is moved past the connector 12c and the Y shaped connector 16 in a forward direction 18. But when the wire 12b is withdrawn from the conduit and moved past the connector 12c back into the sleeve 12f in a direction opposite to direction 18, the distal end of the wire 12b connected to the transducer 12a is then located near the connector 12c as shown in Fig. 1A.

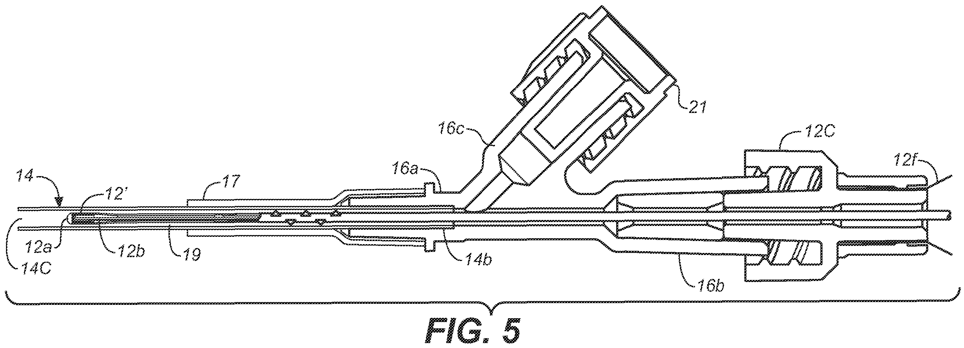

The Y shaped connector 16 has at its proximal end a second port 16b shaped so that the port mates with the connector 12c (shown in Fig. 5) to form a stable and sturdy connection between the artery catheter 14 and the cardiac output Doppler catheter 12. The Doppler transducer 12a in distal end 12' of catheter 12 and the wire 12b are movable relative to port 16a and this stable connection so as to insert or withdraw the Doppler transducer 12a and the wire 12b into or from the conduit in the artery catheter 14.

The sleeve 12f is shorter than the wire 12b so that when the wire is withdrawn from the conduit in the artery catheter 14, the sleeve limits the extent by which the wire is withdrawn from the conduit. Fig. 3 is a cross-sectional view of the distal end 12' of the Doppler catheter that contains the Doppler transducer, and of a cap 22 for protecting the Doppler transducer. As shown in Fig. 3, cap 22 is shaped to mate with the connector 12c when the Doppler catheter is not connected to the artery catheter body, to enclose and protect the Doppler transducer 12a. The connector 12c also centers the Doppler transducer 12a when the Doppler transducer is inserted into the conduit in the body of the artery catheter 14.

Fig. 4 is a cross-sectional view of the Y shaped connector of Figs. 1B where the wire 12b of the Doppler catheter 12 is not in the conduit 14c of the artery catheter As shown in Fig. 4, the proximal end 14b of the artery catheter 14 is inserted into the distal end 16a of the Y shaped connector 16, and a shrink tubing 17 fits snugly by a heat shrink process over the connection between the artery catheter 14 and the Y shaped connector 16 to reduce the stress on and chance of breakage of the artery catheter 14. The Y shaped connector 16 includes a Touchy Borst valve that tightens around the Doppler catheter 12 when longitudinal pressure along direction 18 is applied to prevent blood from leaking past and around the Doppler catheter 12 when it is inserted into the conduit of the artery catheter 14.

Fig. 5 is a cross-sectional view of the Y shaped connector of Figs. 1B, the proximal end 14b of artery catheter 14 and a portion of the Doppler catheter 12 that includes the wire 12b and a portion of sleeve 12f, where the wire 12b of the Doppler catheter 12 is in the conduit 14c of the artery catheter 14. As shown in Fig. 5 (not drawn to scale), conduit 14c has cross-sectional dimensions that are such that there is clearance 19 between the Doppler catheter 12 containing wire 12b and the surface of the conduit 14c. This allows a clear path between the third monitoring/infusion port 16c (also shown in Fig. 4) of connector 16 and the artery into which the artery catheter is placed, even where wire 12b is in the conduit 14c. Thus, even where the Doppler catheter 12 has been inserted and is housed by the conduit 14c, it is still possible to monitor the blood pressure in the artery through the monitoring/infusion port 16c of connector 16 so that the blood pressure of the patient can be monitored while ultrasound measurement of blood mean velocity in the artery is being performed. Of course, where the Doppler catheter 12 is not in the conduit, blood pressure in the artery can be monitored also. The monitoring/infusion port 16c of connector 16 can also be used for infusion or injection of a substance such as crystalloids or hyperalimentation fluids. Thus, blood pressure monitoring, substance infusion or injection, and retrieval of blood samples in or from the artery can also be performed while blood mean velocity is being measured as described above, or when the Doppler catheter is not used for measuring blood mean velocity and wire 12b is not in conduit 14c. These functions may be performed by connecting the appropriate equipment to monitoring/infusion port 16c through a port connector 21 that connects to and fits over port 16c as shown in Fig. 5. When not in use, port 16c and the port at the proximal end 16b of the Y shaped connector 16 may be enclosed by means of connectors 23 as shown in Fig. 4.

Preferably, the elongated catheter body of the artery catheter 14 comprises a material firm enough to enable the body to be inserted into arteries, but flexible enough to bend along turns in the arteries, such as turns in an umbilical artery in newborns. In one embodiment, the elongated catheter body of the artery catheter 14 comprises a polyurethane material. While connector 16 preferably is Y-shaped, it will be understood that connector 16 may be in shapes other than Y; such variations are within the scope of the invention. Preferably the monitoring/infusion port 16c of connector 16 is located between ports 16a and 16b at the distal and proximal ends of the connector respectively.

Fig. 6A is a partly perspective and partly cross-sectional view of the cardiac output Doppler catheter 12 and artery catheter 14 of Fig. 1A and of a connector 16 with the monitoring/infusion port 16c port connected to yet another connector 24 with two ports: a port connected to a syringe 26 and another port connected to a pressure transducer 28 to illustrate applications of one embodiment of the invention. The connector 24 contains a valve (not shown) therein to allow either the syringe 26 or the pressure transducer 28, but not both, to be connected to port 16c at any one time. Part of the cardiac output Doppler catheter 12 is truncated and not shown in Fig. 6A. Fig. 6B is an exploded view of a portion of the Doppler catheter and artery catheter of Fig. 6A within the circle 6B in Fig. 6A. As shown in Fig. 6A. through connectors 16 and 24, the blood pressure in the artery may be monitored by means of a pressure transducer 28 providing a pressure reading at an output. Substances may also be injected into the artery through connectors 16 and 24 by means of syringe 26.

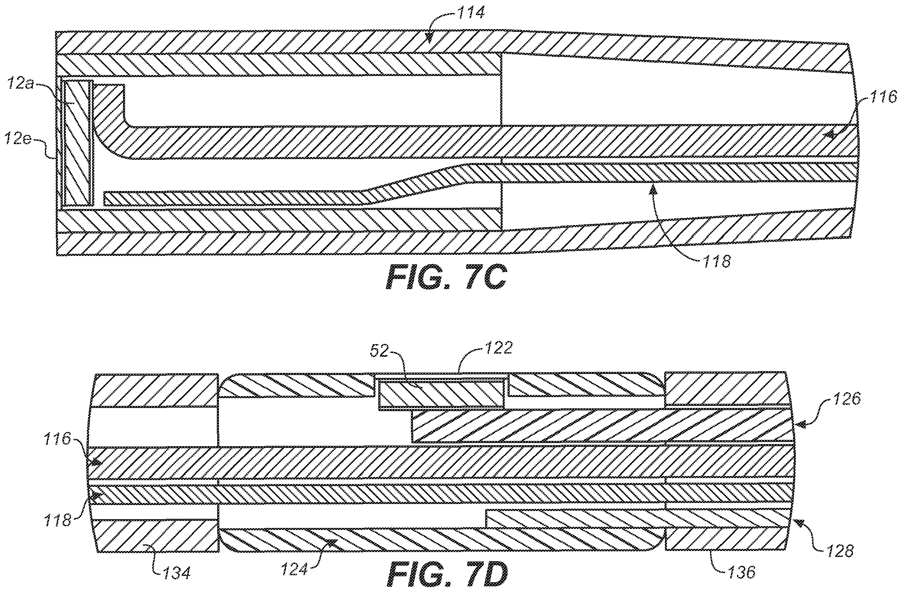

Fig. 7A is a perspective view of components of a portion of a cardiac output Doppler catheter 112 to illustrate an alternative embodiment of the invention with two transducers. Fig. 7B is a cross-sectional view of components of the distal end of the cardiac output Doppler catheter of Fig. 7A after the components are assembled. Figs. 7C, 7D are cross-sectional views of portions of the cardiac output Doppler catheter of Figs. 7A, 7B to illustrate the connections of power and ground leads to the two transducers.

As shown in Fig. 7A instead of having only one Doppler transducer 12a as in the embodiments described above, cardiac output Doppler catheter 112 of Figs. 7A-7E includes at least two transducers: Doppler transducer 12a as in the embodiments described above, but also at least a second transducer 52. Transducers 12a, 52 comprise piezo crystals #1 and #2 respectively as shown in Fig. 7A. Cardiac output Doppler catheter 112 includes a housing 114 for the piezo crystal #1, which is protected by the housing 114 and a matching layer 12e from the environment in the artery. Power lead 116 is electrically connected to piezo crystal #1, and an optional ground lead 118 is preferably electrically connected to housing 114 (connections shown in dashed lines in Fig. 7A). Cardiac output Doppler catheter 112 also includes a housing 124 for the piezo crystal #2, which is protected by the housing 124 and a matching layer 122 from the environment in the artery. Power lead 126 is electrically connected to piezo crystal #2, and an optional ground lead 128 is preferably electrically connected to housing 124 (connections shown in dashed lines in Fig. 7A). The housings 114 and 124 are then connected together by sections 132, 134 of a jacket 1 and section 136 of jacket 2 to form the distal end of cardiac output Doppler catheter 112, where the leads 116, 128, 126, 128 are housed within the sections. Fig. 7B is a cross-sectional view of components of the distal end of the cardiac output Doppler catheter 112 of Fig. 7A after the components are assembled.

Fig. 7C is a cross-sectional view of a portion of the cardiac output Doppler catheter of Fig. 7A, 7B within the circle 7C in Fig. 7B to illustrate the connections of power and ground leads to the Doppler transducer 12a. Fig. 7D is a cross-sectional view of a portion of the cardiac output Doppler catheter of Figs. 7A, 7B within the circle 7D in Fig. 7B to illustrate the connections of power and ground leads to the transducer 52. Aside from employing additional transducers such as transducer 52 together with the associated leads or wires, the alternative embodiment of Figs. 7A-7E uses the same components and functions in the same manner as the embodiments of Figs. 1A through 6B for measuring blood mean velocity.

The advantage of having at least a second transducer that is employed on the side of the Doppler catheter 12 is that this second transducer can be used for measuring a cross-sectional dimension of the artery. This can be done by simply detecting the time of travel of sound waves and its reflection between the second transducer and an adjacent wall of the artery. The distance between the transducer and the artery wall can then be calculated from the time measured and the speed of sound in the blood. If it can be assumed that the Doppler catheter 12 is placed at the central axis of the artery and that the artery has a substantially circular cross-section, then the cross-sectional dimension of the artery can be computed from this time measurement, and from the speed of sound in the blood. If the artery does not have a substantially circular cross-section, employing additional transducers located at different positions around a circumference or perimeter of the Doppler catheter will provide measurement of distances between the Doppler catheter and the artery wall at multiple positions around a circumference or perimeter of the artery to yield multiple (e.g. at least two) cross-sectional dimensions of the artery. Alternatively, instead of using more than one transducer (side transducer) located on the side of Doppler catheter 12, the Doppler catheter 12 may be rotated in the artery so that the only one side transducer is used to measure multiple (e.g. at least two) cross-sectional dimensions of the artery at two or more different angular orientations of the Doppler catheter relative to the artery. More accurate cross-section dimensions of the artery can then be derived from the above measurements.

The ultrasound measurement provided by the Doppler transducer provides the blood mean velocity. Blood volume flow or cardiac output can then be computed from this mean velocity and the cross-sectional dimensions of the artery. Blood pressure monitoring, substance infusion or injection, and retrieval of blood samples in or from the artery can be performed while blood flow is being measured as described above, or when the Doppler catheter is not used for measuring blood flow and the Doppler catheter is not in conduit 14c.

Newborn infants, especially premature infants, often have cardiopulmonary problems at birth. These may be related to lung disease as in respiratory distress syndrome or problems at birth due to congenital deformities or blood loss at birth decreasing blood volume. Compounding this is a patent ductus arteriosus (PDA) which shunts blood between the pulmonary artery and the aorta in both directions. Consequently there is poor oxygenation of the blood or flooding of the lungs - this is typically referred to as a ventilation (V) perfusion (P) mismatch. The direction or quantitative measure of blood flow cannot be obtained continuously with available devices at present. Blood pressure is measured via an umbilical artery catheter, the tip of which is in the aorta, to reflect/approximate blood flow and cardiac output - this is very imprecise and sub optimal in managing sick infants. Further, precise flow or cardiac output display is crucial to appropriately prescribe interventions with medications, ventilation or blood volume to rectify the V/Q mismatch.

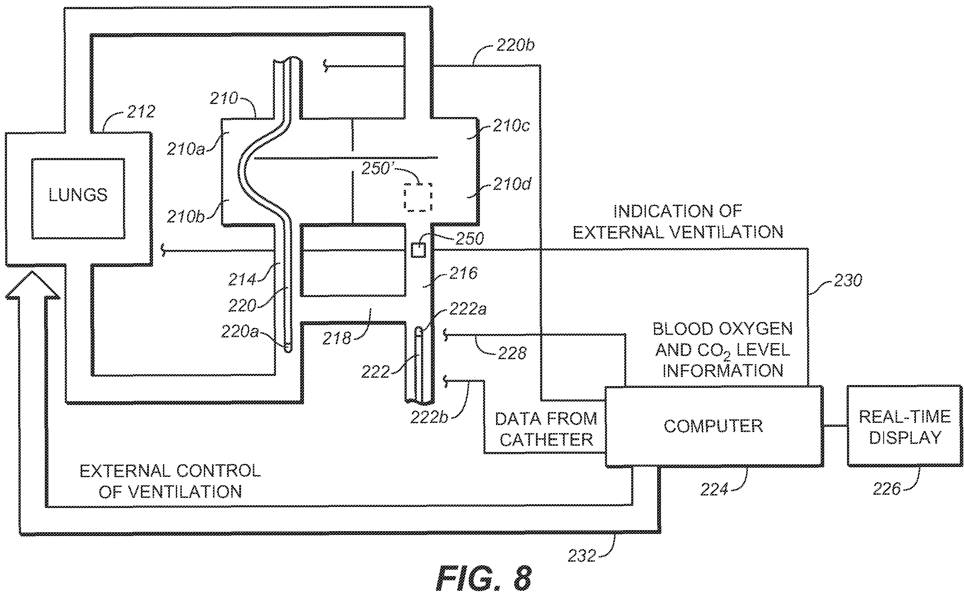

The different embodiments above may be used for measurements and display of information as described in U.S. Pat, 5,433,205. Sections below are taken from this patent to illustrate how the different embodiments above may be used. Fig. 8 of this document contains the same information as Fig. 1 of U.S. Pat, 5,433,205. U.S. Pat, 5,433,205 describes a method of using a Doppler-tipped catheter to produce an indication of blood flow through an artery for determining shunt fractions through the ductus arteriosus of a neonatal patient. The proposed methodology of an embodiment of the invention in this application uses a custom catheter with an indwelling Doppler probe to measure cardiac output or flow, and quantitate the shunt fraction through the PDA of newborn infants, especially premature infants.

FIG. 8 is a schematic view of parts of the body, including the heart 210 and lungs 212. The heart is divided into the right atrium 210a and right ventricle 210b, and the left atrium 210c and left ventricle 210d. The pulmonary artery 214 connects the right ventricle 210b to the lungs 212. The aorta 216 is a passage through which blood goes to the different parts of the body. In a fetus and neonatal patient, the pulmonary artery 214 and aorta 216 are connected by the ductus arteriosus 218.

FIG. 8 shows two positions in which a catheter can be placed into the arteries to determine the blood flow of the patient. Doppler-tipped catheter 220 (which can be any one of the embodiments described above) is positioned down through the heart past the ductus arteriosus in the pulmonary artery 214. Notice that the tip of the Doppler-tipped catheter 220a is near but facing away from the ductus arteriosus 218. Doppler-tipped catheter 222 is placed through the umbilical cord up through the aorta 216 to a position near and facing the ductus arteriosus 218. The velocity of blood in the bloodstream can be determined with the use of a Doppler-tipped catheter in a manner such that the cardiac output or blood flow Q can be computed from the blood mean velocity and the cross-sectional area of the blood vessel. The mean velocity is determined using the Doppler shift in the frequency between the transmitted signal and the received signal. This shift is proportional to the blood velocity.

An important part of monitoring the ventilation perfusion match is monitoring the blood flow in the body. When patients are on life support systems, oxygen is pumped under pressure into the lung 212 at high concentrations. Thus, if the surface area of the lung 212 available for gas exchange is decreased by disease, e.g. pneumonia, the higher concentration of oxygen will provide an adequate amount of oxygen to the body through the available surface area. Sometimes the lung 212 tends to collapse or have poor compliance, as in premature infants. Pressure to deliver the oxygen and open the lungs then becomes essential. However, excess pressure will over-distend the alveoli and squeeze the surrounding blood out, causing an inadequate availability of blood for gas exchange. In this case, due to over-distension, ventilation V is increased; however, due to the squeeze effect, Q is decreased. Catheter 220a will show decreased flow. Conversely, if the pressure is inadequate, the blood supply remains adequate but the gas supply is reduced--normal Q with decreased V. Please note that the concentration of oxygen may be adequate, just that the surface area available for gas exchange is reduced. In the last case, catheter 220a will show normal flow.

Right to left shunting is used loosely with any condition that leads to inadequate oxygenation of the body. Left to right shunting refers to excess blood supply to the lung.

The ductus arteriosus 218 complicates the above picture. If this conduit between the pulmonary artery, the main blood vessel to the lung, and the aorta, the main blood vessel in the body, does not close, the blood takes the path of least resistance and may bypass the lung completely. This effect is caused by excess pressure or over-distension of the lung described above--right to left shunting with V/Q>1. On the other hand, normally the pressure in the aorta is higher than that of the pulmonary artery. Hence, if the ductus is open, the blood will leak back into the lungs--left to right shunting with V/Q<1. Catheter 222a will show retrograde flow and catheter 220a will show increased antegrade flow. This assumes that normal V/Q=1.

The Doppler-tipped catheter placed into an artery of the patient can help determine shunt fractions through the ductus arteriosus 18. The frequency shift signal from the Doppler-tipped catheter will provide the blood flow velocity toward (antegrade) or away from (retrograde) the catheter. The positive portion of the frequency shift signal gives an indication of the antegrade blood flow through the aorta 216 to the body. The negative portion gives an indication of the left-to-right shunting of retrograde blood flow through the ductus arteriosus 218. In this manner, the shunt fraction through the ductus arteriosus 218 can be determined. Alternately, a Doppler-tipped catheter 220 of FIG. 8 will have a retrograde portion of a frequency shift data which corresponds to the blood flow from the right ventricle 210d to the lungs 212, with an antegrade portion which shows the right-to-left shunting through the ductus arteriosus 218. The signal from the velocimeter connected to the catheter can be set to measure blood flow velocity toward (antegrade) or away from (retrograde) the catheter. The software in the computer 224 switches the velocimeter so as to alternately analyze the antegrade and retrograde portion of the signal every 50 milliseconds.

The above sections are essentially taken from U.S. Pat. 5,433,205. The artery into which Doppler catheter 12 and 112 may be placed as described above can be the radial, pulmonary, femoral or umbilical artery. Where the artery is an umbilical artery of a neonatal patient, said location for locating the Doppler transducer 12a is close to the patent ductus arteriosus of the patient, and the ultrasound measurement measures antegrade and retrograde blood velocity. The analysis and computations mentioned above may be performed using computer 224 of Fig. 8. As described above, blood pressure may be monitored while Doppler ultrasound measurements are performed. Thus, both the blood velocity (or blood flow in the case of Doppler catheter 112) and information related to the blood pressure measurement may be displayed at substantially the same time on display 226 in Fig. 8, in real time and continuously if desired.

A cardiac assist device 250 may be placed in the aorta 216 as shown in Fig. 8 or the left ventricle 210d (shown as 250' in dashed lines). If placed in the aorta 216, and the catheter 220a is at the position shown in Fig. 8, catheter 220a may be used as shown in Fig. 8 to measure the velocity of the combined blood flow in the aorta and cardiac assist device. If placed in the left ventricle 210d, catheter 220a may be advanced to a location (not shown) in tandem or within cardiac assist device 250' to measure the blood velocity in the cardiac assist device 250'. If the catheter 220a is to be used in this manner, the elongated catheter body of catheter 220a may comprise a material rigid enough to be placed in tandem or within cardiac assist devices, and can contain metal.

A systolic time interval parameter can also be derived from a result of the Doppler measurement described above. For details of such derivation, please see Ahmed et al., "Systolic Time Intervals as Measures of the Contractile State of the Left Ventricular Myocardium in Man," Journal of the American Heart Association, Circulation, Vol. XLVI, September 1972, pp. 559-571. The derivation from a result of the Doppler measurement mentioned above may be performed using computer 224 of Fig. 8.

The contractility of the heart and diastolic decay of the blood flow can also be derived from a result of the Doppler measurement described above. For details of such derivation, please see Mason DT et al Assessment of Cardiac Contractility " Circulation 1971 Jul;44(1) 47-58 and Milstein J et al "Assessment of Patent Ductus Arteriosus shunting using diastolic pressure analysis" J of Pediatrics Jan.1979; v94, No1 122-126. The derivation from a result of the Doppler measurement mentioned above may be performed using computer 224 of Fig. 8.

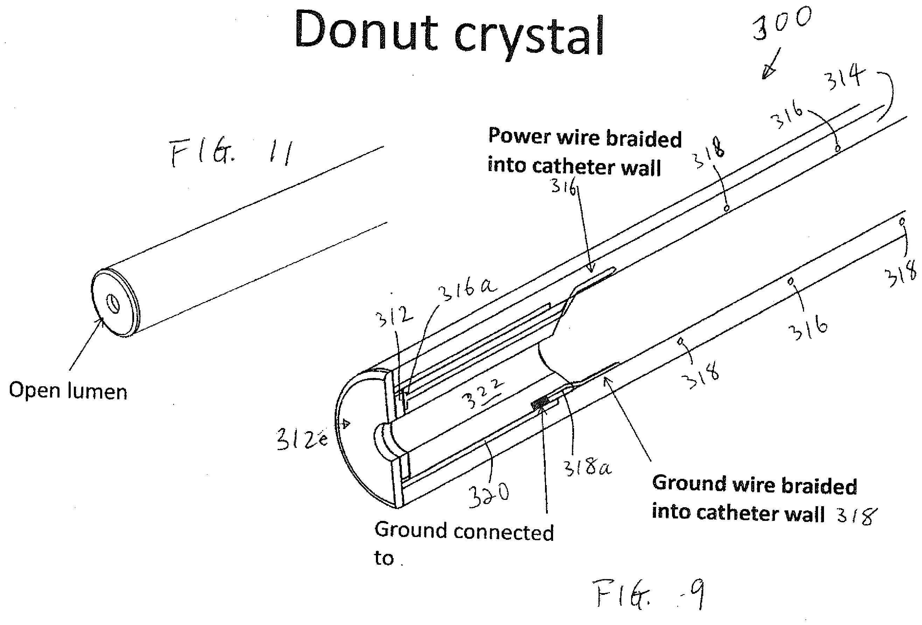

Fig. 9 is a partly perspective and cut away view of the distal end of a Doppler catheter 300 to illustrate another alternative embodiment to the embodiments of this Application. As shown in Fig. 9, Doppler catheter 300 includes a piezo crystal 312, a substantially cylindrical catheter wall 314, power lead 316 and ground lead 318, both leads braided into the catheter wall 314 for driving the piezo crystal 312. Thus, instead of employing power and ground leads that are straight as in Doppler catheters described above, the braided power and ground leads 316, 318 are helical in shape, which render the Doppler catheter 300 more flexible and easier to manipulate around turns in the arteries, such as the turn in an umbilical artery.

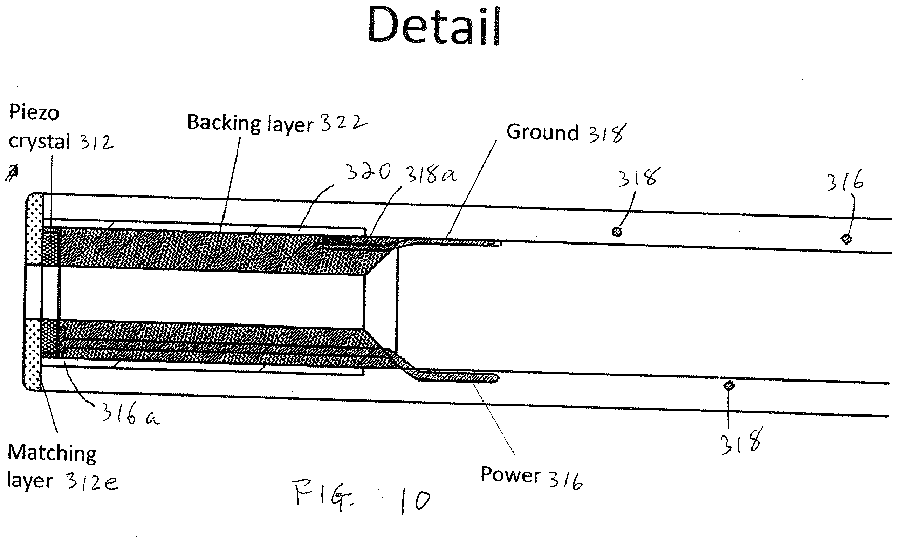

Fig. 10 is a cross-sectional view of Doppler catheter 300 of Fig. 9. As shown in Figs. 9 and 10, end 316a of power lead 316 is electrically and physically connected to the piezo crystal 312, and ground lead 318 is electrically and physically connected to an electrically conductive layer 320 which is in contact with the piezo crystal 312. Thus, when a voltage is applied across the power and ground leads 316, 318, such voltage will cause the piezo crystal 312 to emit ultrasonic waves for Doppler measurements of the artery. The piezo crystal 312 is protected by a Doppler tip matching layer 312e. As shown in Fig. 10, the ends 316a, 318a of the power and ground leads and their connections to the piezo crystal 312 and layer 320 are embedded in a backing layer 322 to ensure that these connections do not become loose after repeated usage of the Doppler catheter 300 over time.

In the embodiments of the Doppler catheter in Figs. 7C and 7D, the ground lead may be connected to the piezo crystal directly or through a connecting layer (not shown in Figs. 7C and 7D), which is in contact with the piezo crystal, similar to the function of layer 320, where such connecting layer forms a part of the housing (e.g. housings 114 and 124 of Fig. 7A).

The piezo crystal 312 and the matching layer 312e have matching holes therein as shown in Figs. 9-11 to provide an additional conduit for blood in the artery. This may increase the accuracy of the blood pressure monitoring, and the ease of substance infusion or injection, and retrieval of blood samples in or from the artery.

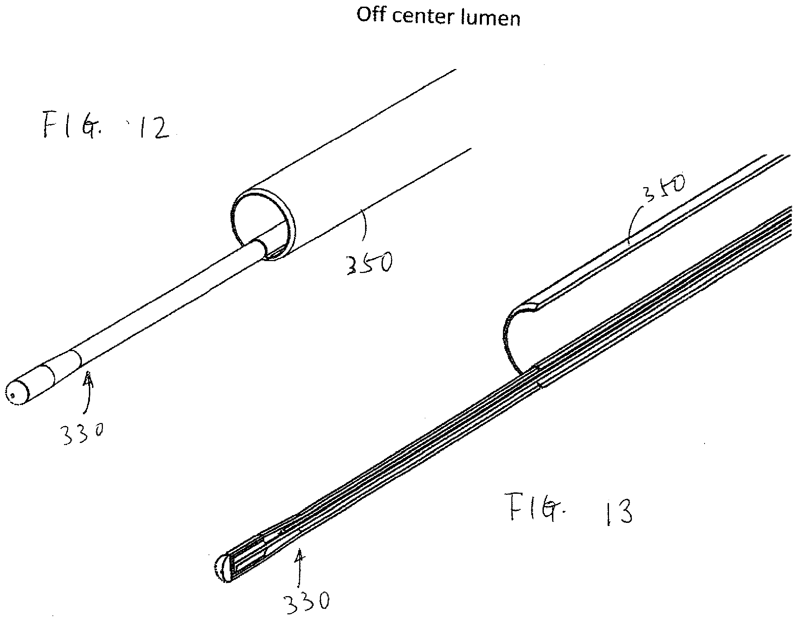

Instead of being centered in the artery catheter, the Doppler catheter 330 may be located at an off-center position within and relative to the artery catheter 350 as shown in Fig. 12. Fig. 13 is a cut away view of the Doppler catheter 330 and the artery catheter 350 of Fig. 12. Catheter 330 may have a construction similar to some of the embodiments of the Doppler catheter with one or more than one piezo crystals described above, such as the Doppler catheter 12 of Fig. 1A or Doppler catheter 112 in Fig. 7A, or may have a construction similar to Doppler catheter 300 of Figs. A, B and C.

As shown in Fig. 13, Doppler catheter 330 has a construction similar to Doppler catheter 300 of Figs. 9-11, except that the power and ground leads are not braided into the catheter wall, but are instead straight. The power and ground leads may be connected to the piezo crystal in a manner similar to that shown in Figs. 9 and 10. The piezo crystal and the matching layer may or may not have matching holes therein.

While the invention has been described above by reference to various embodiments, it will be understood that changes and modifications may be made without departing from the scope of the invention, which is to be defined only by the appended claims.

## Claims

1. An apparatus adapted to perform measurements in an artery, comprising:
a Doppler catheter (12), including a Doppler transducer (12a) and an electrical wire (12b) connected to the Doppler transducer (12a), the Doppler catheter (12) further comprising a first connector (12c) and a second connector (12d), the first connector (12c) containing the electrical wire (12b);
an elongated catheter body (14) having a conduit therein for housing the electrical wire (12b) of the Doppler catheter (12), the elongated catheter body (14) having a proximal end (14b) and a distal end (14a);
a third connector (16) attached to the proximal end (14b) of elongated catheter body (14), the third connector (16) shaped to mate with the first connector (12c) to form a stable connection between the elongated catheter body (14) and the electrical wire (12b); and
a sleeve (12f) connected to the first connector (12c) and the second connector (12d), the sleeve (12f) enclosing the electrical wire (12b) and protecting the electrical wire (12b) from an environment when the electrical wire (12b) is withdrawn from the conduit in the elongated catheter body (14), and wherein the sleeve (12f) is shorter than the electrical wire (12b) such that when the electrical wire (12b) is withdrawn from the conduit in the elongated catheter body (14), the sleeve (12f) limits an extent by which the electrical wire (12b) is withdrawn from the conduit, through the stable connection;
wherein the electrical wire (12b) is movable in the conduit relative to the elongated catheter body (14) so that the Doppler transducer (12a) and the electrical wire (12b) are capable of being threaded into the conduit at the proximal end (14b) through the stable connection after the distal end (14a) of the Doppler catheter (12) has been inserted into the artery, until the Doppler transducer (12a) emerges outside the conduit at the distal end (14a) of the elongated catheter body (14) for performing ultrasound measurements in the artery.

2. The apparatus of claim 1, the Doppler catheter (12) having a distal end and a proximal end, the first connector (12c) being at a location away from the proximal end of the Doppler catheter (12), and the second connector (12d) located at the proximal end of the Doppler catheter (12).

3. The apparatus of claim 1 or 2, wherein the first connector (12c) permits the electrical wire (12b) to be moved relative to the first connector (12c) and to be inserted into, or withdrawn from, the conduit through the stable connection, and wherein when the Doppler transducer (12a) and the electrical wire (12b) are threaded into the conduit at the proximal end (14b) of the elongated catheter body (14) and through the stable connection and moved relative to the first connector (12c) towards the distal end (14a) of the elongated catheter body (14), the Doppler transducer (12a) will emerge at the distal end (14a) of the elongated catheter body (14) and outside the conduit for performing the ultrasound measurements.

4. The apparatus of any of claims 1 to 3, further comprising a cap for connection to the first connector (12c) when the Doppler catheter (12) is not connected to the elongated catheter body (14), to enclose and protect the Doppler transducer (12a).

5. The apparatus of any of claims 1 to 4, wherein the first connector (12c) centers the Doppler transducer (12a) when the Doppler transducer (12a) is inserted into the conduit of the elongated catheter body (14) or the Doppler catheter (12) is in an off-center position in the elongated catheter body (14) after the Doppler transducer (12a) and electrical wire (12b) are inserted into the conduit of the elongated catheter body (14).

6. The apparatus of any of claims 1 to 5, wherein the third connector (16) is attached to the proximal end (14b) of the elongated catheter body (14) and containing a first port that permits the Doppler transducer (12a) and the electrical wire (12b) to pass there through and inserted into the conduit, and a second port connected to the conduit to define a clear path from the second port through the conduit from the distal end (14a) of the elongated catheter body (14) to the proximal end (14b) of the elongated catheter body (14), the clear path enabling blood pressure in the artery to be monitored through the second port whether or not the Doppler transducer (12a) and the electrical wire (12b) are in the conduit, and wherein the third connector (16) having a distal end and a proximal end, a third port at the distal end of the third connector (16) attached to the proximal end (14b) of the elongated catheter body (14), the first port being at a proximal end of the third connector (16) adapted to receive the Doppler transducer (12a) and the electrical wire (12b), and the second port being suitable for infusion of a substance to the artery, for retrieving blood samples, or for blood pressure monitoring.

7. The apparatus of any of claims 1 to 6, wherein the elongated catheter body (14) comprises a material firm enough to enable the elongated catheter body (14) to be inserted into arteries, but flexible enough to bend along turns in an artery as in an umbilical artery in newborns, or wherein the elongated catheter body (14) comprises a material rigid enough to be placed in tandem or within cardiac assist devices.

8. The apparatus of any of claims 1 to 7, wherein the third connector (16) is Y-shaped and the second port is located between the first port and the third port.

9. The apparatus of any of claims 1 to 8, wherein the Doppler catheter (12) further comprises:
at least one second transducer (52) (12a) for measuring at least one cross-sectional dimension of the artery;
at least one second wire connected to the at least one second transducer (52);
at least one ground lead for the Doppler catheter (12) and the at least one second transducer (52); and
a housing containing the electrical wire (12b), the at least one second wire and the at least one ground lead, with the at least one ground lead connected to the housing.

10. The apparatus of any of claims 1 to 9, wherein the Doppler catheter (12) further comprises a catheter wall, wherein the electrical wire (12b) has a helical shape and is embedded in the catheter wall, and a ground lead of helical shape embedded in the catheter wall.

11. The apparatus of any of claims 1 to 10, wherein the artery is an umbilical artery of a neonatal patient, the ultrasound measurements include measuring one or more of antegrade blood velocity, retrograde blood velocity, and derived cardiac indices related to blood velocity in the umbilical artery close to a patent ductus arteriosus of the neonatal patient, the apparatus further comprising a computational element capable of:
a) determining a first cross-sectional measurement of at least one cross-sectional dimension of the umbilical artery by means of the second transducer (52);
b) deriving a blood flow value from a Doppler measurement and the at least one cross-sectional dimension of the umbilical artery;
c) determining a second cross-sectional measurement of the umbilical artery;
d) determining at least two cross-sectional dimensions of the artery at two different angular orientations of the Doppler catheter (12) relative to the umbilical artery; and/or
e) computing one or more of a systolic time interval parameter, contractility of a heart and diastolic decay of blood flow, from a result of the Doppler measurement.

12. The apparatus of any of claims 1 to 11, wherein the artery includes one or more of a radial artery, a pulmonary artery, a femoral artery, or an umbilical artery.

## Patentansprüche

1. Vorrichtung, die angepasst ist, um Messungen in einer Arterie durchzuführen, Folgendes umfassend:
einen Doppler-Katheter (12), der einen Doppler-Wandler (12a) und einen elektrischen Draht (12b), der mit dem Doppler-Wandler (12a) verbunden ist, einschließt, wobei der Doppler-Katheter (12) ferner einen ersten Verbinder (12c) und einen zweiten Verbinder (12d) umfasst, wobei der erste Verbinder (12c) den elektrischen Draht (12b) enthält;
einen länglichen Katheterkörper (14), der in demselben einen Kanal zum Unterbringen des elektrischen Drahtes (12b) des Doppler-Katheters (12) aufweist, wobei der längliche Katheterkörper (14) ein proximales Ende (14b) und ein distales Ende (14a) aufweist;
einen dritten Verbinder (16), der an dem proximalen Ende (14b) des länglichen Katheterkörpers (14) befestigt ist, wobei der dritte Verbinder (16) geformt ist, um mit dem ersten Verbinder (12c) zusammenzupassen, um eine stabile Verbindung zwischen dem länglichen Katheterkörper (14) und dem elektrischen Draht (12b) zu bilden; und
eine Hülse (12f), die mit dem ersten Verbinder (12c) und dem zweiten Verbinder (12d) verbunden ist, wobei die Hülse (12f) den elektrischen Draht (12b) umschließt und den elektrischen Draht (12b) vor einer Umgebung schützt, wenn der elektrische Draht (12b) aus dem Kanal in dem länglichen Katheterkörper (14) herausgezogen wird, und wobei die Hülse (12f) kürzer ist als der elektrische Draht (12b), so dass, wenn der elektrische Draht (12b) aus dem Kanal in dem länglichen Katheterkörper (14) herausgezogen wird, die Hülse (12f), durch die stabile Verbindung, ein Ausmaß begrenzt, um das der elektrische Draht (12b) aus dem Kanal herausgezogen wird;
wobei der elektrische Draht (12b) in dem Kanal derart im Verhältnis zu dem länglichen Katheterkörper (14) beweglich ist, dass der Doppler-Wandler (12a) und der elektrische Draht (12b) dazu in der Lage sind, durch die stabile Verbindung an dem proximalen Ende (14b) in den Kanal gefädelt zu werden, nachdem das distale Ende (14a) des Doppler-Katheters (12) in die Arterie eingeführt worden ist, bis der Doppler-Wandler (12a) an dem distalen Ende (14a) des länglichen Katheterkörpers (14) außerhalb des Kanals auftaucht, zum Durchführen von Ultraschallmessungen in der Arterie.

2. Vorrichtung nach Anspruch 1, wobei der Doppler-Katheter (12) ein distales Ende und ein proximales Ende aufweist, wobei sich der erste Verbinder (12c) an einer Position, entfernt von dem proximalen Ende des Doppler-Katheters (12), befindet und sich der zweite Verbinder (12d) an dem proximalen Ende des Doppler-Katheters (12) befindet.

3. Vorrichtung nach Anspruch 1 oder 2, wobei der erste Verbinder (12c) ermöglicht, dass der elektrische Draht (12b) im Verhältnis zu dem ersten Verbinder (12c) bewegt wird und durch die stabile Verbindung in den Kanal eingeführt oder aus demselben herausgezogen wird, und wobei, wenn der Doppler-Wandler (12a) und der elektrische Draht (12b) an dem proximalen Ende (14b) des länglichen Katheterkörpers (14) in den Kanal und durch die stabile Verbindung gefädelt und im Verhältnis zu dem ersten Verbinder (12c) hin zu dem distalen Ende (14a) des länglichen Katheterkörpers (14) bewegt werden, der Doppler-Wandler (12a) an dem distalen Ende (14a) des länglichen Katheterkörpers (14) und außerhalb des Kanals auftauchen wird, zum Durchführen der Ultraschallmessungen.

4. Vorrichtung nach einem der Ansprüche 1 bis 3, die ferner eine Kappe zur Verbindung mit dem ersten Verbinder (12c) umfasst, wenn der Doppler-Katheter (12) nicht mit dem länglichen Katheterkörper (14) verbunden ist, um den Doppler-Wandler (12a) zu umschließen und zu schützen.

5. Vorrichtung nach einem der Ansprüche 1 bis 4, wobei der erste Verbinder (12c) den Doppler-Wandler (12a) zentriert, wenn der Doppler-Wandler (12a) in den Kanal des länglichen Katheterkörpers (14) eingeführt wird, oder sich der Doppler-Katheter (12) in dem länglichen Katheterkörper (14) in einer exzentrischen Position befindet, nachdem der Doppler-Wandler (12a) und der elektrische Draht (12b) in den Kanal des länglichen Katheterkörpers (14) eingeführt sind.

6. Vorrichtung nach einem der Ansprüche 1 bis 5, wobei der dritte Verbinder (16) an dem proximalen Ende (14b) des länglichen Katheterkörpers (14) befestigt ist und eine erste Öffnung, die ermöglicht, dass der Doppler-Wandler (12a) und der elektrische Draht (12b) durch denselben hindurchgehen und in den Kanal eingeführt werden, und eine zweite Öffnung, die mit dem Kanal verbunden ist, um eine freie Bahn von der zweiten Öffnung durch den Kanal von dem distalen Ende (14a) des länglichen Katheterkörpers (14) bis zu dem proximalen Ende (14b) des länglichen Katheterkörpers (14) zu definieren, enthält, wobei die freie Bahn ermöglicht, dass der Blutdruck in der Arterie durch die zweite Öffnung überwacht wird, ob sich der Doppler-Wandler (12a) und der elektrische Draht (12b) in dem Kanal befindet oder nicht, und
wobei der dritte Verbinder (16) ein distales Ende und ein proximales Ende aufweist, eine dritte Öffnung an dem distalen Ende des dritten Verbinders (16) an dem proximalen Ende (14b) des länglichen Katheterkörpers (14) befestigt ist, die erste Öffnung, die sich an einem proximalen Ende des dritten Verbinders (16) befindet, angepasst ist, um den Doppler-Wandler (12a) und den elektrischen Draht (12b) aufzunehmen, und die zweite Öffnung zur Infusion einer Substanz an die Arterie, zum Gewinnen von Blutproben oder zur Blutdrucküberwachung geeignet ist.

7. Vorrichtung nach einem der Ansprüche 1 bis 6, wobei der längliche Katheterkörper (14) ein Material umfasst, das fest genug ist, um zu ermöglichen, dass der längliche Katheterkörper (14) in Arterien eingeführt wird, aber flexibel genug, um sich entlang von Windungen in einer Arterie, wie in einer Nabelarterie bei Neugeborenen, zu biegen, oder wobei der längliche Katheterkörper (14) ein Material umfasst, das steif genug ist, um im Tandem oder innerhalb von Herzunterstützungsgeräten platziert zu werden.

8. Vorrichtung nach einem der Ansprüche 1 bis 7, wobei der dritte Verbinder (16) Y-förmig ist und die zweite Öffnung zwischen der ersten Öffnung und der dritten Öffnung angeordnet ist.

9. Vorrichtung nach einem der Ansprüche 1 bis 8, wobei der Doppler-Katheter (12) ferner Folgendes umfasst:
mindestens einen zweiten Wandler (52) zum Messen mindestens einer Querschnittsabmessung der Arterie;
mindestens einen zweiten Draht, der mit dem mindestens einen zweiten Wandler (52) verbunden ist;
mindestens eine Masseleitung für den Doppler-Katheter (12) und den mindestens einen zweiten Wandler (52); und
ein Gehäuse, das den elektrischen Draht (12b), den mindestens einen zweiten Draht und die mindestens eine Masseleitung enthält, wobei die mindestens eine Masseleitung mit dem Gehäuse verbunden ist.

10. Vorrichtung nach einem der Ansprüche 1 bis 9, wobei der Doppler-Katheter (12) ferner eine Katheterwand umfasst, wobei der elektrische Draht (12b) eine Wendelform aufweist und in der Katheterwand eingebettet ist und eine Masseleitung mit Wendelform in der Katheterwand eingebettet ist.

11. Vorrichtung nach einem der Ansprüche 1 bis 10, wobei die Arterie eine Nabelarterie eines neugeborenen Patienten ist, die Ultraschallmessungen das Messen eines oder mehrerer von antegrader Blutgeschwindigkeit, retrograder Blutgeschwindigkeit und abgeleiteten Herzkernwerten, die mit der Blutgeschwindigkeit, in der Nabelarterie nahe einem persistierenden Ductus arteriosus des neugeborenen Patienten verbunden sind, einschließen, wobei die Vorrichtung ferner ein rechnergestütztes Element umfasst, das zu Folgendem in der Lage ist:
a) Bestimmen einer ersten Querschnittsmessung mindestens einer Querschnittsabmessung der Nabelarterie mit Hilfe des zweiten Wandlers (52);
b) Ableiten eines Blutflusswertes aus einer Dopplermessung und der mindestens einen Querschnittsabmessung der Nabelarterie;
c) Bestimmen einer zweiten Querschnittsmessung der Nabelarterie;
d) Bestimmen von mindestens zwei Querschnittsabmessungen der Arterie bei zwei unterschiedlichen Winkelausrichtungen des Doppler-Katheters (12) im Verhältnis zu der Nabelarterie; und/oder
e) Berechnen eines oder mehrerer von einem systolischen Zeitintervall-Parameter, einer Kontraktilität eines Herzens und einem diastolischen Abfall des Blutflusses, aus einem Ergebnis der Dopplermessung.

12. Vorrichtung nach einem der Ansprüche 1 bis 11, wobei die Arterie eine oder mehrere von einer Radialarterie, einer Lungenarterie, einer Oberschenkelarterie oder einer Nabelarterie einschließt.

## Revendications

1. Appareil adapté pour effectuer des mesures dans une artère, comprenant :
un cathéter Doppler (12), incluant un transducteur Doppler (12a) et un fil électrique (12b) connecté au transducteur Doppler (12a), le cathéter Doppler (12) comprenant en outre un premier connecteur (12c) et un deuxième connecteur (12d), le premier connecteur (12c) contenant le fil électrique (12b) ;
un corps de cathéter allongé (14) comportant en son sein un conduit pour loger le fil électrique (12b) du cathéter Doppler (12), le corps allongé du cathéter (14) comportant une extrémité proximale (14b) et une extrémité distale (14a) ;
un troisième connecteur (16) fixé à l'extrémité proximale (14b) du corps de cathéter allongé (14), le troisième connecteur (16) étant formé pour s'accoupler avec le premier connecteur (12c) pour former une connexion stable entre le corps allongé du cathéter (14) et le fil électrique (12b) ; et
un manchon (12f) relié au premier connecteur (12c) et au deuxième connecteur (12d), le manchon (12f) renfermant le fil électrique (12b) et protégeant le fil électrique (12b) d'un environnement lorsque le fil électrique (12b) est retiré du conduit dans le corps allongé du cathéter (14), et dans lequel le manchon (12f) est plus court que le fil électrique (12b), de sorte que lorsque le fil électrique (12b) est retiré du conduit dans le corps allongé du cathéter (14), le manchon (12f) limite une étendue sur laquelle le fil électrique (12b) est retiré du conduit à travers la connexion stable ;
dans lequel le fil électrique (12b) peut se déplacer dans le conduit par rapport au corps allongé du cathéter (14) de sorte que le transducteur Doppler (12a) et le fil électrique (12b) peuvent être filetés dans le conduit au niveau de l'extrémité proximale (14b) à travers la connexion stable après l'insertion de l'extrémité distale (14a) du cathéter Doppler (12) dans l'artère, jusqu'à ce que le transducteur Doppler (12a) sorte à l'extérieur du conduit au niveau de l'extrémité distale (14a) du corps allongé du cathéter (14) pour effectuer des mesures ultrasonores dans l'artère.

2. Appareil selon la revendication 1, dans lequel le cathéter Doppler (12) comporte une extrémité distale et une extrémité proximale, le premier connecteur (12c) étant disposé à un emplacement éloigné de l'extrémité proximale du cathéter Doppler (12), et le deuxième connecteur (12d) étant disposé au niveau de l'extrémité proximale du cathéter Doppler (12).

3. Appareil selon les revendications 1 ou 2, dans lequel le premier connecteur (12c) permet le déplacement du fil électrique (12b) par rapport au premier connecteur (12c) et son insertion dans ou son retrait du conduit à travers la connexion stable, et dans lequel, lorsque le transducteur Doppler (12a) et le fil électrique (12b) sont filetés dans le conduit au niveau de l'extrémité proximale (14b) du corps allongé du cathéter (14) et à travers la connexion stable et déplacés par rapport au premier connecteur (12c) vers l'extrémité distale (14a) du corps allongé du cathéter (14), le transducteur Doppler (12a) émergera au niveau de l'extrémité distale (14a) du corps allongé du cathéter (14) et à l'extérieur du conduit pour effectuer les mesures ultrasonores.

4. Appareil selon l'une quelconque des revendications 1 à 3, comprenant en outre un capuchon destiné à être connecté au premier connecteur (12c) lorsque le cathéter Doppler (12) n'est pas connecté au corps allongé du cathéter (14), pour renfermer et protéger le transducteur Doppler (12a).

5. Appareil selon l'une quelconque des revendications 1 à 4, dans lequel le premier connecteur (12c) centre le transducteur Doppler (12a) lorsque le transducteur Doppler (12a) est inséré dans le conduit du corps allongé du cathéter (14) ou lorsque le cathéter Doppler (12) se trouve dans une position décentrée dans le corps allongé du cathéter (14) après l'insertion du transducteur Doppler (12a) et du fil électrique (12b) dans le conduit du corps allongé du cathéter (14).

6. Appareil selon l'une quelconque des revendications 1 à 5, dans lequel le troisième connecteur (16) est fixé sur l'extrémité proximale (14b) du corps allongé du cathéter (14), et contenant un premier orifice qui permet le passage du transducteur Doppler (12a) et du fil électrique (12b) et leur insertion dans le conduit, et un deuxième orifice relié au conduit pour définir un trajet libre depuis le deuxième orifice à travers le conduit depuis l'extrémité distale (14a) du corps allongé du cathéter (14) jusqu'à l'extrémité proximale (14b) du corps allongé du cathéter (14), le trajet libre permettant de surveiller la pression sanguine dans l'artère à travers le deuxième orifice, que le transducteur Doppler (12a) et le fil électrique (12b) soient ou non dans le conduit, et
dans lequel le troisième connecteur (16) comporte une extrémité distale et une extrémité proximale, un troisième orifice au niveau de l'extrémité distale du troisième connecteur (16) fixé à l'extrémité proximale (14b) du corps allongé du cathéter (14), le premier orifice étant situé au niveau d'une extrémité proximale du troisième connecteur (16) adapté pour recevoir le transducteur Doppler (12a) et le fil électrique (12b), et le deuxième orifice étant approprié pour la perfusion d'une substance dans l'artère, pour le prélèvement d'échantillons de sang ou pour la surveillance de la tension artérielle

7. Appareil selon l'une quelconque des revendications 1 à 6, dans lequel le corps allongé du cathéter (14) comprend un matériau suffisamment ferme pour permettre l'insertion du corps allongé du cathéter (14) dans des artères, mais suffisamment flexible pour se plier le long des tournants dans une artère, comme dans une artère ombilicale chez les nouveau-nés, ou dans lequel le corps allongé du cathéter (14) comprend un matériau suffisamment rigide pour être placé en tandem ou dans des dispositifs d'assistance cardiaque .

8. Appareil selon l'une quelconque des revendications 1 à 7, dans lequel le troisième connecteur (16) a une forme en Y et le deuxième orifice est disposé entre le premier orifice et le troisième orifice.

9. Appareil selon l'une quelconque des revendications 1 à 8, dans lequel le cathéter Doppler (12) comprend en outre :
au moins un deuxième transducteur (52) pour mesurer au moins une dimension de section transversale de l'artère ;
au moins un deuxième fil connecté audit au moins un deuxième transducteur (52) ;
au moins un fil de masse pour le cathéter Doppler (12) et l'au moins un deuxième transducteur (52) ; et
un boîtier contenant le fil électrique (12), l'au moins un deuxième fil et l'au moins un conducteur de masse, l'au moins un conducteur de masse étant connecté au boîtier.

10. Appareil selon l'une quelconque des revendications 1 à 9, dans lequel le cathéter Doppler (12) comprend en outre une paroi de cathéter, dans lequel le fil électrique (12b) a une forme hélicoïdale et est noyé dans la paroi du cathéter, et un conducteur de masse de forme hélicoïdale noyé dans la paroi du cathéter.

11. Appareil selon l'une quelconque des revendications 1 à 10, dans lequel l'artère est une artère ombilicale d'un patient nouveau-né, les mesures ultrasonores incluant la mesure d'un ou de plusieurs de la vitesse sanguine antérograde, de vitesse sanguine rétrograde, et des indices cardiaques dérivés concernant la vitesse sanguine dans l'artère ombilicale proche d'un canal artériel persistant du patient nouveau-né, l'appareil comprenant en outre un élément de calcul capable de :
a) déterminer une première mesure en coupe transversale d'au moins une dimension en coupe transversale de l'artère ombilicale au moyen du deuxième transducteur (52) ;
b) dériver une valeur de débit sanguin à partir d'une mesure Doppler et de l'au moins une dimension en coupe transversale de l'artère ombilicale ;
c) déterminer une deuxième mesure en coupe transversale de l'artère ombilicale ;
d) déterminer au moins deux dimensions en coupe transversale de l'artère à deux orientations angulaires différentes du cathéter Doppler (12) par rapport à l'artère ombilicale ; et/ou
e) calculer un ou plusieurs parmi un paramètre d'intervalle de temps systolique, la contractilité d'un coeur et la décroissance diastolique du flux sanguin, à partir d'un résultat de la mesure Doppler.

12. Appareil selon l'une quelconque des revendications 1 à 11, dans lequel l'artère inclut une ou plusieurs d'une artère radiale, d'une artère pulmonaire, d'une artère fémorale ou d'une artère ombilicale.
